# EUROPÄISCHE PATENTSCHRIFT

(11) **EP 1 013 614 B1**
(45) Veröffentlichungstag und Bekanntmachung des Hinweises auf die Patenterteilung: **21.05.2003**
(21) Anmeldenummer: 98811253.8
(22) Anmeldetag: 21.12.1998
(51) Int. Cl.: C02F 11/12, B01D 29/11

(54) **Entwässerung von Fermentationsprodukten mit Schneckenpresse**
Dewatering of fermentation products with screw press
Déshydratation des produits fermentés avec presse à vis sans fin

(43) Veröffentlichungstag der Anmeldung: 28.06.2000
(73) Patentinhaber: Walter Schmid AG, 8152 Glattbrugg (CH)
(72) Erfinder: Schmid, Walter, 8152 Opfikon (CH)
(74) Vertreter: Patentanwälte Feldmann & Partner AG

(56) Entgegenhaltungen:
- EP-A- 0 628 526
- FR-A- 2 138 968
- US-A- 3 938 434
- US-A- 4 834 878
- US-A- 5 122 263
- US-A- 5 827 432

## Beschreibung

Die vorliegende Erfindung betrifft ein Entwässerungsverfahren für Fermentationsprodukte gemäss Oberbegriff des Patentanspruches 1 und eine Vorrichtung zur Durchführung des Verfahrens gemäss Oberbegriff des Patentanspruches 8.

Fermenter zur Vergärung von biogenen Siedlungsabfällen für die Gewinnung von Biogas und Fermentationsprodukten, werden von der Anmelderin entwickelt und seit mehreren Jahren erfolgreich betrieben und verkauft. Der Vergärungsbehälter des Fermenters wird dabei mit dem zu vergärenden organischen Material beschickt und je nach Konsistenz und Wassergehalt mit überschüssigem Presswasser befeuchtet und angeimpft. Um im Fermenter optimale Bedingungen für die an der Vergärung beteiligten Mikroorganismen zu schaffen wird neben anderen Verfahrensparametern vorteilhafterweise der optimale Wassergehalt überwacht und reguliert. Das Material wird durch den Fermenter gefördert und verlässt diesen in vergorenem Zustand als Nass-Gärkompost oder Digestat. Der Trockensubstanzanteil des Nass-Gärkompost kann zwischen ca. 15 bis 30%, typischerweise 22 bis 27%, liegen. Da das Ausgangsmaterial der Fermentation hauptsächlich aus grob zerkleinerten biogenen Haus-, Garten- und Gewerbeabfällen besteht, können im Fermentationsprodukt noch Partikel von bis zu 15 cm Länge und/oder Durchmesser enthalten sein. So können zum Beispiel Tannen- oder Fichtenzapfen, Teile von Aesten und Wurzeln, Steinobstkerne oder auch Knochen und Steine Digestat enthalten sein.
Typische Fermenter weisen ein Volumenfassungsvermögen von bis zu 1500 m³ auf und erzielen einen Durchsatz an zu vergärendem Material von bis zu 18000 t/Jahr. Dem Nass-Gärkompost wird in einer nachgeschalteten Entwässerungsstufe mit Pressen ein Grossteil des Wassers entzogen. Erst dieser entwässerte Feucht-Gärkompost ist für die aerobe Nachrotte geeignet. Das Presswasser weist, obwohl sein Name auf eine Flüssigkeit hinweist, einen relativ hohen Trockensubstanzgehalt auf, ist also dickflüssig und schlammartig. Wie schon oben erwähnt, kann ein Teil des Presswassers zum Animpfen und Befeuchten in den Gärreaktor zurückgeführt werden. Ein weiterer Teil des Presswassers kann bei Bedarf zum Befeuchten der Nachrotte eingesetzt werden. Der Grossteil des verbleibenden Presswassers kann jedoch nicht direkt wiederverwendet werden und muss deshalb entsorgt werden.

Dieses überschüssige Presswasser ist stark mit organischem Material belastet und weist einen hohen Anteil an mineralischen und biogenen Feststoffen auf. Der Trockensubstanzanteil beträgt im allgemeinen von 8% bis 15%, in extremen Fällen von bis zu 20%. Das Ueberschusswassers kann daher in der Regel nicht direkt der Kanalisation zugeführt werden, da die organischen und bakteriellen Verunreinigungen über den Toleranzwerten liegen, die von Kläranlagen akzeptiert werden. Zudem würde der hohe Feststoffanteil zu Sedimentation in der Kanalisation und zu damit verbundenen Problemen führen. Eine vorgängige Behandlung des Ueberschusswassers ist deshalb notwendig.
Diese Problematik tritt in gleicher Weise bei anderen Typen von biologischen Vergärungsanlagen auf. Auch dort existieren Verfahrensstufen, in denen nasse Gärprodukte anfallen und entwässert werden müssen.
Bisher hat man sich mit unbefriedigenden Lösungen begnügt. So wurde zum Beispiel das Presswasser mit Flockungshilfsmittel geflockt und anschliessend getrocknet. Von der Anmelderin ist ein Verfahren bekannt, bei dem der Nass-Gärkompost in einem ersten Schritt in einer Schneckenpresse unter relativ hohem Druck gepresst wird, und das Ueberschusswasser anschliessend in einem mehrstufigen Verfahren aufbereitet wird. In diesem Verfahren werden nach dem Pressen mittels eines Dekanters die im Ueberschusswasser vorhandenen Feststoffe von der flüssigen Phase getrennt. Flockungshilfsmittel unterstützen dabei das Trennverfahren im Dekanter. Die im Dekanter abgeschiedenen Feststoffe bilden ein feinkörniges Material, das in einem weiteren Arbeitschritt wieder mit dem Feucht-Gärkompost vermischt werden muss. Bei der Entwässerung in der Schneckenpresse wird der Gärkompost stark verdichtet, was ihn zu einem suboptimalen Ausgangsmaterial für die Nachrotte macht. Um das Presswasser soweit von organischen Verunreinigungen und von Feststoffen zu befreien, dass eine problemlose Einleitung in die Kanalisation und eine anschliessende Klärung möglich ist, sind also mindestens je eine leistungsstarke und kostenintensive Presse und Zentrifuge/Dekanter nötig. Pressen und Entwässern müssen in getrennten Arbeitsschritten erfolgen und die jeweiligen festen Produkte müssen anschliessend wieder möglichst homogen vermischt werden.

In der US-A-5'122'263 ist ein Entwässerer für Klärschlamm beschrieben, der einen Schneckenförderer mit einem ersten zylindrischen, einem anschliessenden konischen und einem zweiten zylindrischen Bereich umfasst. Im konischen Bereich reduziert sich das Volumen der Entwässerungseinrichtung reduziert sich in Förderrichtung. Im anschliessenden zylindrischen Bereich mit kleinerem Durchmesser sind mehrere Pressbereiche vorhenden. Die Pressbereiche weisen jeweils eingangsseitig bei konstantem Durchmesser der Welle eine abnehmende Steigung der Förderwendel auf. Im Endbereich der Pressbereiche sitzt ein Konuskörper auf der Welle auf. Die Spaltbreite in den zylindrischen Bereichen beträgt 1 bis 0.5 mm, im konischen Bereich etwa 0.25 mm und in den Pressbereichen zwischen 0.25 und 0.1 mm. In der beschriebenen Vorrichtung können Schlämme entwässert werden, welche einen Trockenmassengehalt von 3 bis 7 % aufweisen.

Die FR-A-2'138'968 beschreibt eine Vorrichtung zur kontinuierlichen Entwässerung von Suspensionen mit geringem Gehalt an Feststoffen, insbesondere von chemischen Abwasserschlämmen welche vorgängig mit Ausflockungsagenzien behandelt sind. Für die Trennung von flüssiger und fester Phase ist eine Endlos-Spindelpresse vorgesehen, bei der die Schnecke über ihre gesamte Länge in mehrere zylindrische Schneckenenabschnitte mit stufenweise abnehmendem Durchmesser eingeteilt ist. In den einzelnen Abschnitten bleiben Gangbreite und Gangtiefe der Schnecke konstant. An mehreren Stellen ist die Schnecke unterbrochen, und die Schneckenachse wird von Stauringen umgeben, welche an der Siebwand befestigt sind. Die Stauringe reduzieren die Kanaltiefe und sollen die Bildung eines Filterkuchens an der Siebwand reduzieren und durch das Zurückdrängen der zu trennenden Materialsuspension für eine bessere Trennleistung sorgen. Entladungsmündung durch einen Druckkegel geschlossen werden kann, der von Außenseite gegen die Mündung gedrückt werden kann. Dem Entwässerer vorgeschaltet ist ein Mischer, in dem Flockungsmittel den zu entwässernden Schlämmen zugegeben werden.

Es ist die Aufgabe der Erfindung, ein Verfahren und eine Vorrichtung zur Entwässerung von Nass-Gärkompost zur Verfügung zu stellen, welche die oben genannten Nachteile nicht aufweisen.

Diese Aufgabe lösen ein Verfahren mit den Merkmalen des Patentanspruches 1 und eine Vorrichtung mit den Merkmalen des Patentanspruches 9.

In der beiliegenden Zeichnung sind zwei Ausführungsbeispiele der erfindungsgemässen Vorrichtung dargestellt, die in der nachfolgenden Beschreibung in Zusammenhang mit dem erfindungsgemässen Verfahren erläutert werden. Es zeigen
- Figur 1: eine schematische Seitenansicht einer Entwässerungsanlage; und
- Figur 2: einen schematischen Querschnitt einer Variante des Entwässerers, wobei das Gehäuse weggelassen ist.

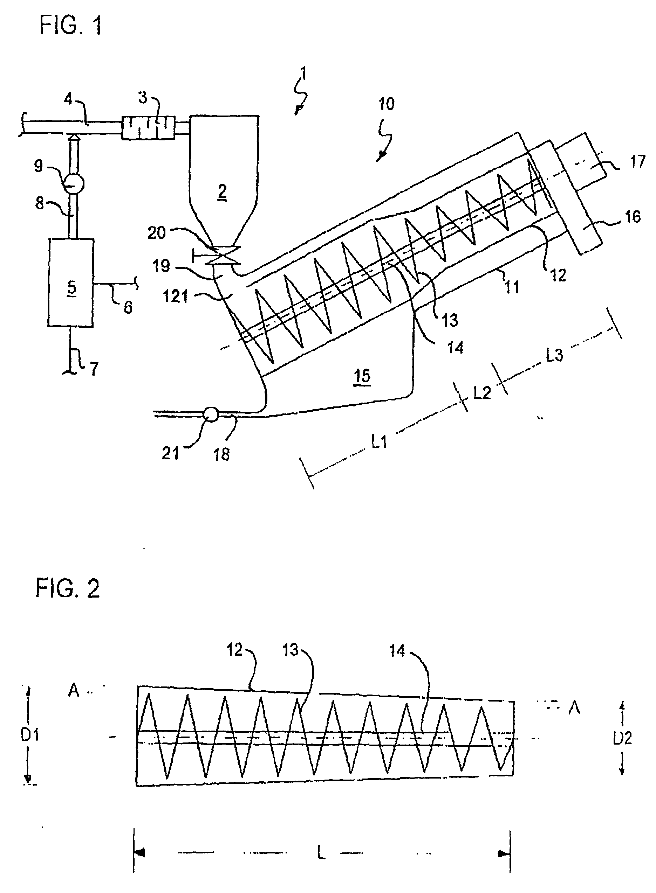

Wie in der Figur 1 skizziert, wird in der erfindungsgemässen Entwässerungsanlage 1 dem aus einem nicht dargestellten Fermenter stammenden Nass-Gärkompost (auch Fermentationsprodukt oder Digestat) über eine Speiseleitung 8 mittels einer Dosierpumpe 9 Flockungshilfsmittel F zugegeben. Die Aufbereitung des handelsüblichen Flockungshilfsmittels F erfolgt im Flockungshilfsmittelbereiter 5, dem Wasser über die Leitung 6 und Flockungshilfsmittelkonzentrat über die Zuführleitungen 7 aus hier nicht dargestellten Speichertanks oder Leitungen zugeführt werden.
Der Nass-Gärkompost weist einen Trockensubstanzanteil von ca. 15 bis 30% auf. Er wird üblicherweise diskontinuierlich, das heisst schubweise, aus dem Fermenter über die Zuleitung 4 in die Entwässerungsanlage 1 geleitet. Die Steuerung des Flockungshilfsmittelbereiters 5 und vor allem die Zugabe des Flockungshilfsmittels ist auf diese diskontinuierliche Nass-Gärkompost-Zufuhr angepasst. Dadurch ist sichergestellt, dass der Anteil Flockungshilfsmittel pro Tonne Trockensubstanz des Gärkompostes in einem optimalen Bereich von ca. 0 bis 4 kg, vorzugsweise 1 bis 4 kg konstant gehalten wird. Nass-Gärkompost und Flockungshilfsmittel werden in einer anschliessenden Mischstrecke 3 miteinander vermischt. Die Mischstrecke umfasst einen statischen Mischer 3 mit Umwälzblechen. Der Mischstrecke 3 ist ein Zwischenspeicher 2 nachgeschaltet, in dem die Mischung aus Nass-Gärkompost und Flockungshilfsmittel bis zur Abgabe in den Entwässerer 10 verbleibt. Die Zwischenlagerung ermöglicht einerseits den kontinuierlichen Betrieb des nachgeschalteten Entwässerers 10 und andererseits unterstützt die Verweilzeit im Tank 2 die Wirkung des Flockungshilfsmittels.

Der Entwässerer 10 kann in Abhängigkeit von der örtlichen Raumsituation mehr oder weniger stark geneigt sein. Ist die Entwässerungsanlage zum Beispiel in einem Gebäude untergebracht, so kann im Gegensatz zu der in Figur 1 dargestellten geneigten Version mit einem liegenden Entwässerer 10 die Bauhöhe des Gebäudes niedrig gehalten werden. Für den Fachmann ist es offensichtlich, dass zu diesem Zwecke dann auch die Zuführung der Nass-Gärkompost und Flockungshilfsmittel-Mischung in den Entwässerer 10 anders als in der Figur 1 dargestellt gelöst wird. Der Zwischenspeicher 2 wird dazu vorzugsweise sehr flach gestaltet, oder gänzlich neben dem Entwässerer 10 plaziert.
Zwischen dem Speichertank 2 und dem Entwässerer 10 ist ein steuerbarer Sperrschieber 20 plaziert, mittels dessen die Zufuhr von Nass-Gärkompost zum Entwässerer 10 dosiert und bei Bedarf ganz unerbrochen werden kann.
Auf den Zwischenspeicher 2 kann auch ganz verzichtet werden, sofern der Betrieb des Entwässerers 10 dem diskontinuierlichen Ausstoss von Nass-Gärkompost aus dem Fermenter angepasst wird. In einer weiteren bevorzugten, hier jedoch nicht dargestellten Ausführungsform der Entwässerungsanlage 1 ist die Zuleitung zum Enwässerer 10 so gestaltet, dass die Mischung aus Nass-Gärkompost und Flockungshilfsmittel wahlweise in den Zwischenspeicher 2 oder direkt in den Entwässerer 10 geleitet werden kann.

Ein erfindungsgemässer zylindrischer bis kegelstumpfförmiger Entwässerer 10, der mit Erfolg in einer Pilotanlage betrieben wird hat ein Fassungsvermögen von ca. 2.5 m³. Bei einer Durchlaufzeit der Mischung aus Nass-Gärkompost und Flockungshilfsmittel von 5 bis 20 Minuten, vorzugsweise von 10 bis 15 Minuten, ergibt sich ein ungefährer stündlicher Durchsatz von 6 m³.

Wie in Figur 1 dargestellt ist im Gehäuse 11 des Entwässerers 10 auf der Achse 14 eine Schnecke 13 rotierbar angeordnet. Die Schnecke 13 ist in ihrer gesamten Länge von einem koaxial feststehenden Siebkorb oder Sieb 12 umschlossen.

Die Durchmesser des Siebes 12 und der darin gelagerten Schnecke 13 sind, wie in den Figuren 1 und 2 dargestellt, üblicherweise nicht konstant, sondern verjüngen sich über die Länge L des Entwässerers 10. Die in der Figur 2 angedeutete Verjüngung von Siebdurchmesser (D1 zu D2) und Schneckendurchmesser erfolgt in gleichem Masse, so dass das Schneckenspiel A über die gesamte Länge L des Entwässerers annähernd konstant bleibt.
In der Figur 1 ist eine weitere Variante dargestellt, in der auf einen ersten zylindrischen Abschnitt L1 ein konischer Abschnitt L2 und dann wiederum ein zylindrischer Abschnitt L3 folgt.
Um der schwierigen Stoffcharakteristik des Nass-Gärkompostes, insbesondere den grossen Partikeln von bis zu 10 cm Durchmesser, gerecht zu werden weist das Sieb 12 des Entwässerers 10 der Pilotanlage einen eintrittsseitigen Durchmesser von 100 cm auf. Der Abstand zwischen Sieb 12 und
Schnecke 13 beträgt 15 cm, das Schneckenspiel demnach 30 cm. Dies entspricht einem Verhältnis von Durchmesser/Schneckenspiel von 0.3.
Die Siebflächen 12 sind vorzugsweise aus Edelstahl gefertigt, so dass sie vom alkalischen Nass-Gärkompost nicht angegriffen werden und eine optimale Lebensdauer bei geringem Wartungsaufwand gewährleistet ist. Da das zu entwässernde Material kontinuierlich entlang der Siebflächen 12 gefördert wird, baut sich kein Filterkuchen auf und es kommt während des Betriebes zu keinem ungewünschten lokalen Anstieg des Filtrationswiderstandes. Ueber die gesamte Länge des Entwässerers 10 kommt es durch die Verjüngung von Schraube 13 und Sieb 12 in Kombination mit einer Abnahme der Lochweite im Sieb 12 und der langsamen Fördergeschwindigkeit im Entwässerer 10 zu einer kontinuierlichen Entwässerung des Nass-Gärkompostes. Die Schneckengeometrie, die Form des Siebes 12 und die Lochweite des Siebes 12 sind so aufeinander abgestimmt, dass es zu einem kontinuierlichen sanften Druckanstieg in Förderrichtung kommt. Dadurch wird über die gesamte Länge eine gute Entwässerungsleistung gewährleistet. Der Aufbau von hohen Drücken wird gleichzeitig vermieden und ein Teil der Entwässerungsleistung ist schon allein durch das passive schwerkraftgetriebene Abtropfen des Gärkompostes im Zwischenspeicher 2 und im Entwässerer 10 gewährleistet.

Diese schonende verdichtungsarme Entwässerung hat mehrere gewünschte Folgen. Zum einen wird die Wirkung des Flockungshilfsmittles nicht durch hohe Drücke beeinträchtigt, zum anderen genügt ein verhältnissmässig schwacher Schneckenantrieb 17 zum Betrieb des Entwässerers 10. Es kann also der Energieeinsatz zum Entwässern niedrig gehalten werden, und die gesamte Konstruktion muss keinen hohen mechanischen Belastungen standhalten. Zusätzlich bleibt das Digestat durch die kontinuierliche Umwälzung bei der Förderung im Entwässerer locker und wird nicht in unerwünschter Weise verdichtet, was sich wiederum positiv auf die Nachrotte auswirkt.
Das austretende Filtrat mit einem Trockensubstanzanteil von ca. 1% sammelt sich im Sumpf 15 und kann über die Ableitung 18 direkt der Kanalisation zugeführt werden. Je nach Bedarf wird dazu eine Filtratpumpe 21 eingesetzt.
Der entwässerte Gärkompost mit einem Trockensubstanzanteil von ca. 38 bis 45 % wird über den Auswurf 16 ausgestossen und kann ohne weitere Bearbeitung der Nachrotte zugeführt werden.
Der Begriff verdichtungsarm wird hier verwendet um deutlich hervorzuheben, dass es sich nicht primär um eine Entwässerung durch Raumverdrängung handelt, sondern um eine sehr geringe Verdichtung die lediglich zu einer besseren Agglomeration der Feuchtigkeitspartikel führen soll und dann zu einem Abtropfen. Entwässerer ähnlicher Bauart sind bekannt, haben jedoch eine stärkere Konizität, die entsprechend zu einer Pressung mit hoher Verdichtung führt und wiederum eines hohen Energieeinsatzes bedarf.
Die oben beschreibene erfindungsgemässe Vorrichtung respektive das erfindungsgemässe Verfahren erlauben es unter verwendung der Flockungshilfsmittel den Trockensubstanzanteil des Ueberschusswassers von bisher üblichen Werten zwischen 10 und 15% auf unter 3 bis 4% zu senken. Wird auf solche Flockungshilfsmittel verzichtet, so kann der Trockensubstanzanteil im Ueberschusswasser immer noch auf unter 8% gehalten werden.
Um den Wartungsaufwand niedrig zu halten und einen möglichst kontinuierlichen Betrieb zu gewährleisten, kann der Entwässerer mit einer automatischen Rückspüleinrichtung für das Sieb 12 ausgestatten sein.

## Patentansprüche

1. Verfahren zur Entwässerung eines Fermentationsproduktes aus biogenen Abfallstoffen **dadurch gekennzeichnet, dass**
a) das Fermentationsprodukt kontinuierlich in einem Schneckenentwässerer (10) verdichtungsarm entwässert wird, wobei im Gehäuse (11) des Entwässerers (10) auf einer Achse (14) eine Schnecke (13) rotierbar angeordnet ist und die Schnecke (13) in ihrer gesamten Länge von einem koaxial feststehenden Sieb (12) umschlossen ist und sich Sieb (12) und Schnecke (13) über die Länge L des Entwässerers (10) verjüngen, und die Lochweite im Sieb (12) über die Länge L abnimmt, und
b) das Fermentationsprodukt mit einer Durchlaufzeit von 5 bis 20 Minuten, vorzugsweise 10 bis 15 Minuten, den Entwässerer durchläuft,
wobei das Spiel der Schnecke 13) zwischen 25 und 35%, vorzugsweise 30% des Durchmessers des Siebes (12) beträgt.

2. Verfahren nach Anspruch 1, **dadurch gekennzeichnet, dass** dem zu entwässernden Fermentationsprodukt vor der Zuführung zum Schneckenentwässerer ein Flockungshilfsmittel zudosiert wird, und das Fermentationsprodukt und das Flockungshilfsmittel miteinander vermischt werden.

3. Verfahren nach Anspruch 2, **dadurch gekennzeichnet, dass** die Mischung aus Fermentationsprodukt und Flockungshilfsmittel in einem Tank (2) zwischengelagert wird, bevor sie dem Entwässerer (10) zugeführt wird.

4. Verfahren nach einem der Ansprüche 1 bis 3, **dadurch gekennzeichnet, dass** das Fermentationsprodukt Partikel von bis zu 15 cm Länge aufweist.

5. Verfahren nach Anspruch 4, **dadurch gekennzeichnet, dass** das Fermentationsprodukt ein Nass-Gärkompost oder Digestat mit einem Trockensubstanzanteil von 15 bis 30 %, vorzugsweise 22 bis 27 %, ist.

6. Verfahren nach einem der Ansprüche 1 bis 5, **dadurch gekennzeichnet, dass** der Trockensubsanzanteil des entwässerten Fermentationsprodukts zwischen 38 und 45 % , vorzugsweise zwischen 39 und 41 % liegt.

7. Verfahren nach Anspruch 1 oder 2, **dadurch gekennzeichnet, dass** pro Tonne Trockensubstanz des Fermentationsproduktes zwischen 0 und 4 kg, vorzugsweise 1 bis 4 kg Flockungshilfsmittel zudosiert werden.

8. Verfahren nach Anspruch 3, **dadurch gekennzeichnet, dass** die Anlieferung des Fermentationsproduktes und darauf abgestimmt die Zugabe des Flockungshilfsmittels zum Tank (2) diskontinuierlich erfolgt.

9. Anlage zur Durchführung eines Entwässerungsverfahrens insbesondere nach einem der Ansprüche 1 bis 8 mit einem Schneckenentwässerer (10) in dessen Gehäuse (11) auf einer Achse (14) eine Schnecke (13) rotierbar angeordnet ist, wobei die Schnecke (13) in ihrer gesamten Länge von einem koaxial feststehenden Sieb (12) umschlossen ist, Sieb (12) und Schnecke (13) sich über die Länge L des Entwässerers (10) verjüngen, und die Lochweite im Sieb (12) über die Länge L abnimmt, und dem Entwässerer (10) eine Mischstrecke (3) vorgeschaltet ist, die über Zuleitungen (9) mit einem Fermenter und einem Flockungshilfs-mittelbereiter (5) verbunden ist, **dadurch gekennzeichnet, dass** das Spiel der Schnecke (13) zwischen 25 und 35%, vorzugsweise 30% des Durchmessers des Siebes (12) beträgt.

10. Anlage nach Anspruch 9, **dadurch gekennzeichnet, dass** zwischen Mischstrecke (3) und Entwässerer (10) ein Zwischenspeicher (2) angeordnet ist.

11. Anlage nach einem der Ansprüche 9 oder 10, **dadurch gekennzeichnet, dass** sich Sieb (12) und Schnecke (13) über die Länge L des Entwässerers (10) verjüngen.

12. Anlage nach Anspruch 9, **dadurch gekennzeichnet, dass** die Mischstrecke (3) einen statischen Mischer (3)umfasst.

13. Anlage nach einem der Ansprüche 9 bis 12, **dadurch gekennzeichnet, dass** sie eine automatische Spüleinrichtung zum Reinigen und/oder Rückspülen des Siebes (12) aufweist.

## Revendications

1. Procédé pour la déshydratation d'un produit de fermentation de substances résiduaires biogènes, **caractérisé en ce que**
a) la produit de fermentation est déshydraté en continu dans un dispositif d'assèchement à vis sans fin (10) à faible compression, tandis que dans le carter (11) du dispositif d'assèchement (10) une vis sans fin (13) est disposée avec rotation sur un axe (14) et que la vis sans fin (13) est entourée sur toute sa longueur par un tamis fixe coaxial (12) et le tamis (12) et la vis sans fin (13) se rétrécissent sur la longueur L du dispositif d'assèchement (10), et le diamètre des trous dans le tamis (12) va en diminuant sur la longueur L, et
b) le produit de fermentation parcourt le dispositif d'assèchement avec une durée de passage de 5 à 20 minutes, de préférence de 10 à 15 minutes,
tandis que le pas de la vis sans fin (13) vaut entre 25 et 35 %, de préférence 30 % du diamètre du tamis (12).

2. Procédé selon la revendication 1, **caractérisé en ce qu'**un adjuvant de floculation est ajouté an quantité dosée au produit de fermentation à déshydrater, avant l'introduction dans le dispositif d'assèchement à vis sans fin, et que le produit de fermentation et l'adjuvant de floculation sont mélangés ensemble.

3. Procédé selon la revendication 2, **caractérisé en ce que** le mélange du produit de fermentation et de l'adjuvant de floculation est entreposé dans un réservoir (2), avant d'être introduit dans le dispositif d'assèchement (10).

4. Procédé selon l'une des revendications 1 à 3, **caractérisé en ce que** le produit de fermentation présente des particules ayant une longueur allant jusqu'à 15 cm.

5. Procédé selon la revendication 4, **caractérisé en ce que** le produit de fermentation est un compost de fermentation humide ou un produit de digestion ayant une proportion de substance sèche de 15 à 30 %, de préférence de 22 à 27 %.

6. Procédé selon l'une des revendication 1 à 5, **caractérisé en ce que** la proportion de substance sèche du produit de fermentation déshydraté se situe entre 38 et 45 %, de préférence entre 39 et 41 %.

7. Procédé selon la revendication 1 ou 2, **caractérisé en ce que** par tonne de substance sèche du produit de fermentation sont ajoutés en quantité dosée entre 0 et 4 kg, de préférence 1 à 4 kg d'adjuvant de floculation.

8. Procédé selon la revendication 3, **caractérisé en ce que** la fourniture du produit de fermentation et l'addition en quantité correspondante de l'adjuvant de floculation dans le réservoir (2) est effectuée en discontinu.

9. Installation pour la mise en oeuvre d'un procédé de déshydratation en particulier selon l'une des revendications 1 à 8 avec un dispositif d'assèchement à vis sans fin (10) dans le carter (11) duquel est disposée en rotation sur un axe (14) une vis sans fin (13), tandis que la vis sans fin (13) est entourée sur toute sa longueur par un tamis fixe coaxial (12), le tamis (12) et la vis sans fin (13) se rétrécissent sur la longueur L du dispositif d'assèchement (10), et le diamètre des trous dans le tamis (12) va en diminuant sur la longueur L, et est disposée en tête du dispositif d'assèchement (10) une section de mélange (3) qui est reliée par l'intermédiaire de conduites d'alimentation (9) à un fermenteur et à un dispositif (5) de préparation d'adjuvant de floculation, **caractérisée en ce que** le pas de la vis sans fin (13) vaut entre 25 et 35 %, de préférence 30 % du diamètre du tamis (12).

10. Installation selon la revendication 9, **caractérisée en ce qu'**un silo-tampon (2) est disposé entre la section de mélange (3) et le dispositif d'assèchement (10).

11. Installation selon l'une des revendication 9 ou 10, **caractérisée en ce que** le tamis (12) et la vis sans fin (13) se rétrécissent sur la longueur (L) du dispositif d'assèchement (10).

12. Installation selon la revendication 9, **caractérisée en ce que** la section de mélange (3) comporte un mélangeur statistique (3).

13. Installation selon l'une des revendications 9 à 12, **caractérisée en ce qu'**elle présente un dispositif de rinçage automatique pour le nettoyage et/ou le lavage à contre-courant du tamis (12).

## Claims

1. A method for draining a fermentation product from biogenic waste substances, **characterised in that**
a) the fermentation product is drained continuously in a low compression manner in a worm drainer (10), wherein in the housing (11) of the drainer (10) a worm (13) is rotatably arranged on a shaft (14) and the worm (13) is enclosed over its whole length by a coaxial stationary sieve (12) and the sieve (12) and the worm (13) taper over the length L of the drainer (10), and the hole width in the sieve (12) reduces over the length L, and
b) the fermentation product runs through the drainer with a run-through time of 5 to 20 minutes, preferably 10 to 15 minutes,
wherein the play of the worm (13) is between 25 and 35%, preferably 30% of the diameter of the sieve (12).

2. A method according to claim 1, **characterised in that** a flocculent is metered to the fermentation product to be drained, before feeding to the worm drainer, and the fermentation product and the flocculent are mixed with one another.

3. A method according to claim 2, **characterised in that** the mixture of fermentation product and flocculent are intermediately stored in a tank (2) before it is supplied to the drainer (10).

4. A method according to one of claims 1 to 3, **characterised in that** the fermentation product comprises particles of up to 15 cm length.

5. A method according to claim 4, **characterised in that** the fermentation product is a wet ferment-compost or digestate with a dry substance component of 15 to 30 %, preferably 22 to 27 %.

6. A method according to one of the claims 1 to 5, **characterised in that** the dry substance component of the fermentation product to be drained lies between 38 % and 45 %, preferably between 39 and 41 %.

7. A method according to claims 1 or 2, **characterised in that** per ton of dry substance of the fermentation product one meters between 0 and 4 kg, preferably 1 to 4 kg of flocculent.

8. A method according to claim 3, **characterised in that** the delivery of the fermentation product and the addition of flocculent to the tank (2) being matched to this fermentation product is effected discontinously.

9. An installation for carrying out a draining method, in particular according to one of the claims 1 to 8 with a worm drainer (10) in whose housing (11) a worm (13) is rotatably arranged on a shaft (14), wherein the worm (13) is enclosed over its whole length by a coaxial stationary sieve (12) and the sieve (12) and the worm (13) taper over the length L of the drainer (10), and the hole width in the sieve (12) reduces over the length L, and a mixing stretch (3) is arranged upstream of the drainer (1), which via feed conduits (9) is connected to a flocculent preparer (5), **characterised in that** the play of the worm (13) is between 25 and 35 %, preferably 30 % of the diameter of the sieve (12).

10. An installation according to claim 9, **characterised in that** an intermediate storer (2) is arranged between the mixing stretch (3) and the drainer (10)

11. An installation according to one of the claims 9 or 10, **characterised in that** the sieve (12) and the worm (13) taper over the length L of the drainer (10).

12. An installation according to claim 9, **characterised in that** the mixing stretch (3) comprises a static mixer (3).

13. An installation according to one of the claims 9 to 12, **characterised in that** it comprises an automatic rinsing means for cleaning and/or return rinsing of the sieve (12).
